# EUROPÄISCHE PATENTSCHRIFT

(11) **EP 1 415 675 B1**
(45) Veröffentlichungstag und Bekanntmachung des Hinweises auf die Patenterteilung: **17.05.2006**
(21) Anmeldenummer: 03024763.9
(22) Anmeldetag: 29.10.2003
(51) Int. Cl.: A61M 5/168

(54) **Ventil zum Einstellen des Volumenstromes bei einem Infusions- oder Transfusionsvorgang**
Flow control valve for infusion or transfusion purposes
Valve pour contrôler le débit dans une procédé de perfusion où de transfusion

(30) Priorität: 29.10.2002 DE 10250391
(43) Veröffentlichungstag der Anmeldung: 06.05.2004
(73) Patentinhaber: Smiths Medical Deutschland GmbH, 85614 Kirchseeon (DE)
(72) Erfinder: Beck, Bernd, 72414 Rangendingen (DE); Weber, Jörg, 83533 Edling (DE)
(74) Vertreter: von Bülow, Tam

(56) Entgegenhaltungen:
- EP-A- 0 123 079
- EP-A- 0 552 854
- US-A- 5 113 904

## Beschreibung

Die Erfindung bezieht sich auf ein Regelventil gemäß dem Oberbegriff des Patentanspruches 1.

Ein solches Regelventil ist aus der DE 83 12 029 U bekannt. Dieses Regelventil hat ein topfförmiges Gehäuse mit einem Einlaß- und einem Auslaßanschlußstutzen, die in einen hohlzylindrischen Ventilraum münden und ein Drehteil mit einem topfförmigen Grundkörper und einem davon abstehenden zylindrischen Ventilküken, das außenseitig dichtend in den Ventilraum einsetzbar ist und an seinem Außenumfang mindestens eine den Anschlußstutzen gegenüberliegenden Nut aufweist, die die Anschlußstutzen strömungsmäßig miteinander verbindet und in Umfangsrichtung einen sich ändernden Querschnitt aufweist.

Ein ähnliches Regelventil ist in der DE 43 40 191 C1 beschrieben. Zur Drehbegrenzung des Drehteiles sind dort am Boden des topfförmigen Gehäuses sowie am Boden des topfförmigen Drehkörpers komplementäre Anschläge für eine Drehbegrenzung vorgesehen.

Die DE 42 01 416 A1 hat einen Drehkörper mit zwei in Axialrichtung gegeneinander versetzt angeordnete Ringnuten, die durch einen axial verlaufenden Kanal verbunden sind. Damit können Einlaß- und Auslaßanschluß in Axialrichtung gegeneinander versetzt sein.

Ein weiteres ähnliches Regelventil ist in der DE 35 90 339 C2 beschrieben, wobei dort ein zylinderförmiger Drehkörper an seiner Stirnseite eine sich in Umfangsrichtung verbreiternde Nut hat und Einlaß- und Auslaßanschlüsse in den Boden eines topfförmigen Gehäuses münden. Voraussetzung für eine einwandfreie Funktion ist dort ein dichtender Kontakt zwischen Boden des Gehäuses und Stirnfläche des Drehkörpers, da ansonsten eine Strömungsverbindung zwischen Einlaß- und Auslaßanschluß außerhalb der Nut möglich ist.

Die JP 2002035123 A zeigt eine Rechenscheibe zur Berechnung der Durchflußrate bei einer Tropfinfusion. Auf einer ebenen Platte sind mehrere Skalen auf konzentrischen Kreisen aufgebracht. Eine darüberliegende Drehscheibe hat entsprechend den Skalen mehrere kreissegmentförmige Fenster, durch welche hindurch die Skalen sichtbar sind.

Die Durchflußrate bei solchen Regelventilen hängt nicht nur vom wirksamen Querschnitt sondern auch vom hydrostatischen Druck ab. Üblicherweise wird ein Gefäß mit der Infusionsflüssigkeit an ein Infusionsständer befestigt, wobei das Gefäß in vorgegebener Höhe an dem Ständer angebracht wird. Eine übliche Höhe ist beispielsweise 50 cm oberhalb der Infusionsstelle am Patienten. Eine Veränderung dieser Höhe führt auch zu einer Veränderung der Tropfrate.

Wünschenswert wäre es für das Klinikpersonal eine Aussage über die Tropfrate in Abhängigkeit vom hydrostatischen Druck und in Abhängigkeit von der Drehstellung des Drehkörpers zu erhalten, und zwar so, daß die Auswirkung einer Änderung des hydrostatischen Druckes, also der Höhe des Behälters, schon vor Veränderung der Höhe abgeschätzt werden kann. Daneben soll das Regelventil einfach aufgebaut und zu montieren, einfach, d.h. möglichst mit einer Hand medienbar sein und eine präzise Einstellung der Durchflußrate ermöglichen.

Ebenso wird bei bekannten Durchflußreglern häufig beanstandet, daß nur die Flußraten für eine niederviskose Kochsalzlösung angezeigt werden. Bei Verwendung höherviskoser Flüssigkeiten sind die üblichen Skalen nicht anwendbar, ja sogar irreführend und können zu Fehlinterpretationen führen. Demgegenüber wäre eine Skala zur Angabe der Flußrate höherviskoser Flüssigkeiten für den Anwender von Vorteil.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung liegt darin, im Deckel des Drehkörpers mehrere Öffnungen bzw. Fenster vorzusehen, die um den Umfang verteilt und radial gegeneinander versetzt angeordnet sind. Innerhalb des Gehäuses und dabei unterhalb dieser Fenster sind mehrere Markierungen bzw. Skalen vorgesehen, nämlich entsprechend der Anzahl der Fenster, auf denen die Durchflußrate ablesbar ist.

Das Problem höherviskoser Flüssigkeiten läßt sich durch eine der Skalen lösen, die beispielsweise lediglich eine einfache Kennzeichnung mit Buchstaben hat, wobei entsprechende Referenzwerte den einzelnen Buchstaben zugeordnet sind und in einer beigefügten Bedienungsanleitung, einem Handbuch o.ä. entsprechenden Referenzwerten zugeordnet werden können. Eine andere Skala ist eine Standardskala für isotonische Kochsalzlösungen.

Im folgenden wird die Erfindung anhand eines Ausführungsbeispieles im Zusammenhang mit der Zeichnung ausführlicher erläutert. Es zeigt:
- Fig. 1: eine Explosionszeichnung eines Regelventiles nach der Erfindung;
- Fig. 2: das Gehäuse des Regelventiles in Ansicht von unten (Fig. 2a), zwei Seitenansichten (Fig. 2b und 2c) sowie zwei Schnittansichten (Fig. 2d und 2e);
- Fig. 3: verschiedene Ansichten eines Einsatzkörpers in Draufsicht (Fig. 3a), zwei Seitenansichten (Fig. 3b und 3d) und zwei Schnittansichten (Fig. 3c und 3e);
- Fig. 4: verschiedene Ansichten des Drehkörpers, nämlich eine Draufsicht (Fig. 4a), zwei Seitenansichten (Fig. 4b und 4d) sowie zwei Schnittansichten (Fig. 4c und 4e);
- Fig. 5: einen Querschnitt des Regelventiles in zusammengebautem Zustand;
- Fig. 6: zwei Schnitte durch den Drehkörper im Bereich der Ringnut als Querschnitt (Fig. 6a) und Längsschnitt (Fig. 6b);
- Fig. 7: eine Explosionszeichnung eines Regelventiles nach einem zweiten Ausführungsbeispiel der Erfindung;
- Fig. 8: verschiedene Darstellungen und Ansichten des Gehäuses des Ausführungsbeispieles der Fig. 7;
- Fig. 9: verschiedene Darstellungen und Ansichten des Ventilkörpers des Ausführungsbeispieles der Fig. 7;
- Fig. 10: verschiedene Darstellungen und Ansichten des Drehteiles des Ausführungsbeispieles der Fig. 7; und
- Fig. 11: verschiedene Ansichten und Darstellungen des zusammengesetzten Regelventils des Ausführungsbeispieles der Fig. 7.

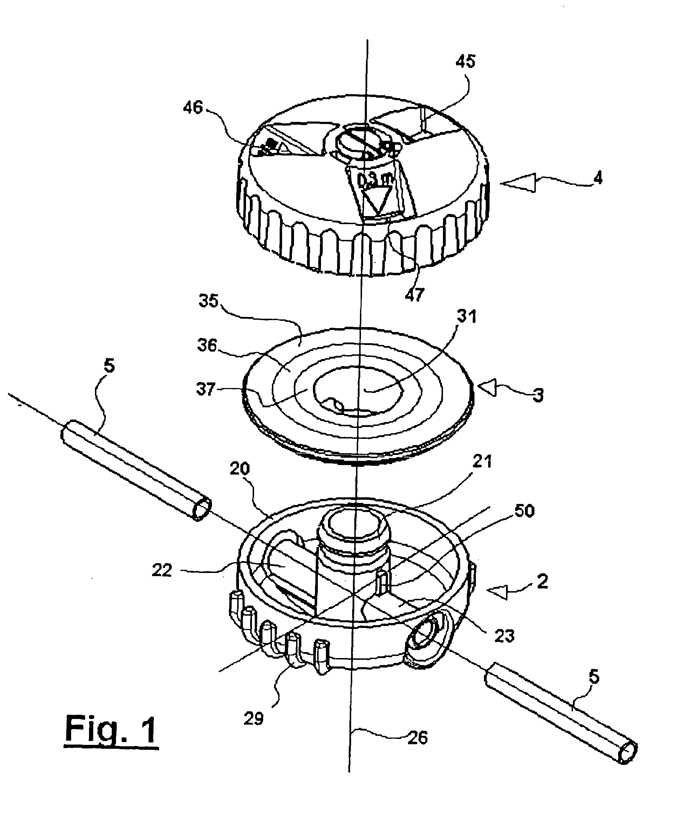

Zunächst sei auf Fig. 1 Bezug genommen. Das Regelventil, das in seiner Gesamtheit mit dem Bezugszeichen 1 bezeichnet ist, hat ein Gehäuse 2, einen Einsatzkörper 3 und ein Drehteil 4. Über zwei Schlauchenden 5 wird das Regelventil mit einem nichtdargestellten Behälter für Infusionsflüssigkeit und einem Blutgefäß des Patienten verbunden.

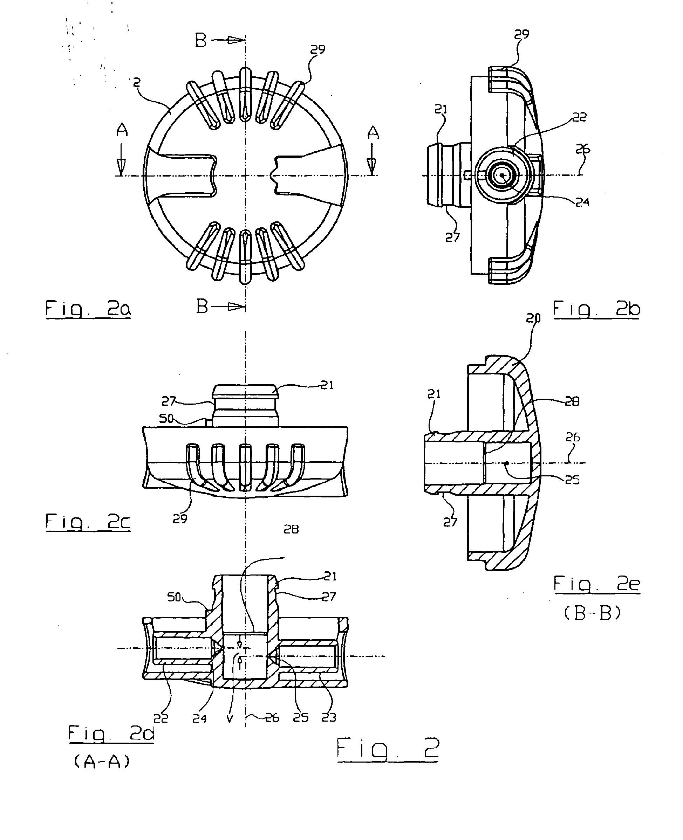

Das Gehäuse 2 hat einen topfförmigen Grundkörper 20, an den ein hohlzylindrischer, kaminartiger Ventilraum 21 angeformt ist, sowie zwei seitliche Anschlußstutzen 22 und 23 für die Schläuche (5 in Fig. 1), die über Öffnungen 24 bzw. 25 mit dem Innenraum des zylindrischen Ventilraumes 21 verbunden sind. Die beiden Anschlußstutzen 22 und 23 sind bezogen auf die Längsachse 26 des Ventilraumes 21 gegeneinander versetzt angeordnet (vgl. Fig. 2d) und liegen sich in Umfangsrichtung um 180° versetzt gegenüber. Am Außenumfang des topfförmigen Grundkörpers 20 sind mehrere Griffnoppen 29 angeformt, die ein festes, verdrehsicheres Halten mit der Hand gestatten.

Weiter ist ein ringscheibenförmiger Einsatz 3 mit einer mittigen Öffnung 31 vorgesehen, die den Ventilraum 21 übergreift, d.h. der Einsatz 3 wird über den Ventilraum 21 geschoben. An seiner dem Gehäuse abgewandten Oberseite sind auf den Einsatz 3 auf mehreren konzentrischen Kreisbahnen Markierungen 35, 36 und 37 aufgebracht. Der Einsatz 3 ist gegenüber dem Gehäuse 2 eindeutig fixiert, wie noch aus der späteren Beschreibung hervorgeht.

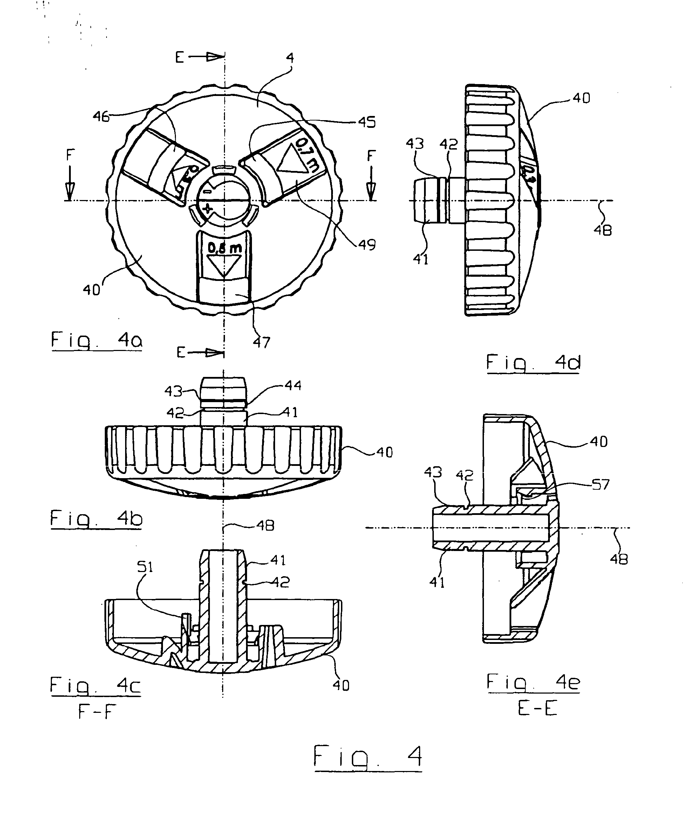

Schließlich hat das Regelventil ein Drehteil 4, welches, wie im Zusammenhang mit Fig. 4 noch ausführlicher erläutert wird, ein topfförmiges Gehäuse und ein davon abstehendes hohlzylindrisches Ventilküken 41 aufweist, das in den Ventilraum 21 eingreift. Die Außenseite des Drehteiles 4 hat mehrere Fenster 45, 46 und 47, die in Umfangsrichtung gleichmäßig verteilt sind und die in Radialrichtung jeweils zueinander versetzt angeordnet sind. Jedes Fenster 45, 46 und 47 ist einer Markierung 35, 36, 37 zugeordnet, so daß die entsprechende Markierung durch das zugeordnete Fenster ablesbar ist.

Das Regelventil 1 nach dem ersten Ausführungsbeispiel besteht im Prinzip also nur aus drei Teilen 2, 3, 4, die beispielsweise aus Kunststoff im Spritzgußverfahren hergestellt werden. Die drei Teile sind sehr einfach zu montieren. Es wird lediglich der Einsatz 3 aufgesetzt und dann das Drehteil 4 darüber geschoben und durch einen weiter unten noch beschriebenen Schnappverschluß verriegelt.

Fig. 2 zeigt verschiedene Ansichten des Gehäuses 2. es hat einen topfförmigen Grundkörper 20, von dem mittig der hohlzylindrische Ventilraum 21 absteht. An die Außenseite des Ventilraumes 21 sind die beiden Anschlußstutzen 22 und 23 angeformt, die über Öffnungen 24 bzw. 25 in Strömungsverbindung mit dem Innenraum des Ventilraumes 21 stehen. Die beiden Anschlußstutzen 22 und 23 sind - bezogen auf die Drehachse 26 des Regelventiles - um eine Strecke V gegeneinander versetzt. An der Außenseite des freien Endes des Ventilraumes 21 ist eine Rastvertiefung 27 vorgesehen, in die eine Rastnase des Drehteiles eingreift. Im Übergangsbereich zwischen dem einen Anschlußstutzen 22 und dem Ventilraum 21 ist an der Außenfläche des Ventilraumes 21 ein radialer Vorsprung vorgesehen, der als Drehbegrenzungsanschlag 50 dient.

In den Schnittdarstellungen der Fig. 2b und 2d ist im Innenraum des Ventilraumes 21 eine Kante 28 zu erkennen, ab der das Ventilküken (Fig. 4) in radial dichtendem Eingriff mit dem Ventilraum 21 steht.

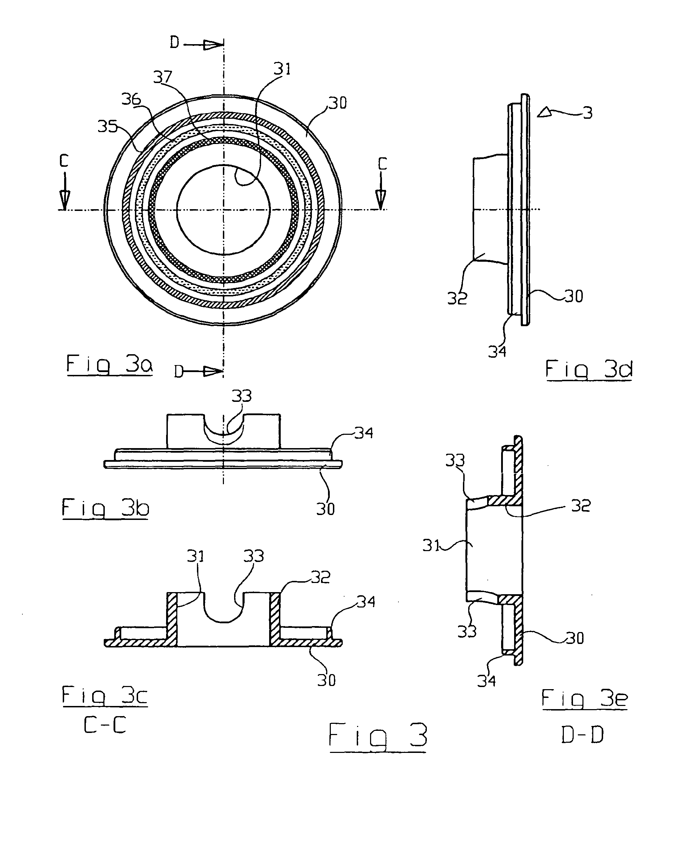

Fig. 3 zeigt den Einsatz 3, der eine ebene Kreisringscheibe 30 mit einer Öffnung 31 bildet, rings um die ein zylindrischer kaminartiger Vorsprung 32 absteht. Der Vorsprung 32 hat an seinem freien Ende zwei gegenüberliegende Ausnehmungen 33 mit halbkreisförmigem Grund, die die Außenseiten der Anschlußstutzen 22 und 23 (aus Fig. 2) übergreifen, womit der Einsatz 3 verdrehsicher im Gehäuse 2 gehalten ist. Weiter hat die Kreisringscheibe 30 nahe dem Außenumfang einen Zentrierbund 34 zur Zentrierung gegenüber dem Gehäuse 2. Dieser Zentrierbund steht von der Kreisringscheibe 30 in derselben Richtung ab wie der Vorsprung 32.

Auf der in Fig. 3a sichtbaren Oberseite der Kreisringscheibe 30 sind auf konzentrischen Ringen Markierungen bzw. Skalen 35, 36 und 37 aufgebracht, beispielsweise in Form von Zahlenwerten für Tropfraten in Abhängigkeit von der Drehstellung des Drehteiles (4 in Fig. 1), wobei die radial gegeneinander versetzt angeordneten Zahlenwerte für unterschiedliche hydrostatische Drücke bzw. Höhen eines Behälters mit Infusionsflüssigkeit gelten. Eine andere Skala kann beispielsweise Buchstaben aufweisen, die Viskositätswerten der Flüssigkeit zugeordnet sind.

Aus den Figuren 3b, 3c und 3e ist zu erkennen, daß die einander gegenüberliegenden Ausnehmungen 33 unterschiedliche Tiefe haben, nämlich entsprechend dem axialen Versatz V der Anschlußstutzen 22 und 23 (Fig. 2).

Fig. 4 zeigt das Drehteil 4, das ebenfalls einen topfförmigen Grundkörper und ein davon abstehendes hohlzylindrisches Ventilküken 41 aufweist. Das Ventilküken 41 hat an seinem Außenumfang zwei Ringnuten 42 und 43, die in Axialrichtung gegeneinander versetzt sind. Die eine Nut 42 hat konstante Tiefe und konstanten Querschnitt, während die andere Nut 43 in Umfangsrichtung einen sich stetig ändernden Querschnitt, nämlich eine sich stetig ändernde Tiefe aufweist und in Umfangsrichtung einen begrenzten Bereich überstreicht, der hier beispielsweise bei 330° liegt. Am Anfang der Nut 43, nämlich dort, wo diese ihre größte Tiefe hat, ist ein axialer Kanal 44 vorgesehen, der die beiden Nuten 42 und 43 miteinander verbindet.

Der Grundkörper 40 hat, wie am besten aus Fig. 4a zu erkennen ist, mehrere Fenster 45, 46 und 47, die jeweils um 120° in Umfangsrichtung versetzt angeordnet sind und gegenüber der Drehachse 26 auch radial versetzt angeordnet sind, so daß sie sich nicht überlappen. Benachbart zu den Fenstern 45, 46 und 47 sind jeweils noch Vertiefungen 49 vorgesehen, in denen eine Markierung aufgebracht ist, wie z.B. eine Bedruckung, die unterschiedliche Höhen angibt, beispielsweise 0,5 m, 0,3 m und 0,7 m. Weiter ist dort ein Ablesepfeil zu erkennen, der dann auf die Markierung der darunterliegenden Markierung der Kreisringscheibe 30 verweist.

Wie am besten aus Fig. 4e zu erkennen ist, hat der Grundkörper 40 konzentrisch zu dem Veritilküken 41 einen Vorsprung 57 mit einem Rasthaken, der mit der Rastvertiefung 27 des Gehäuses (Fig. 2) zusammenwirkt und das Drehteil mit dem Gehäuse nach der Montage fest verbindet.

Zur Drehbegrenzung ist am Ventilraum 21 des Gehäuses 2 oberhalb des Anschlußstutzens 22 ein Drehbegrenzungsanschlag 50 vorgesehen, der mit einem Drehbegrenzungsanschlag 51 am Drehteil 4 zusammenwirkt. Die Drehbegrenzungsanschläge 51 und 52 sind in Umfangsrichtung so angeordnet und dimensioniert, daß der volle Wirkungsbereich der Nut 43 durchlaufen werden kann, also hier beispielsweise der Bereich von 330°.

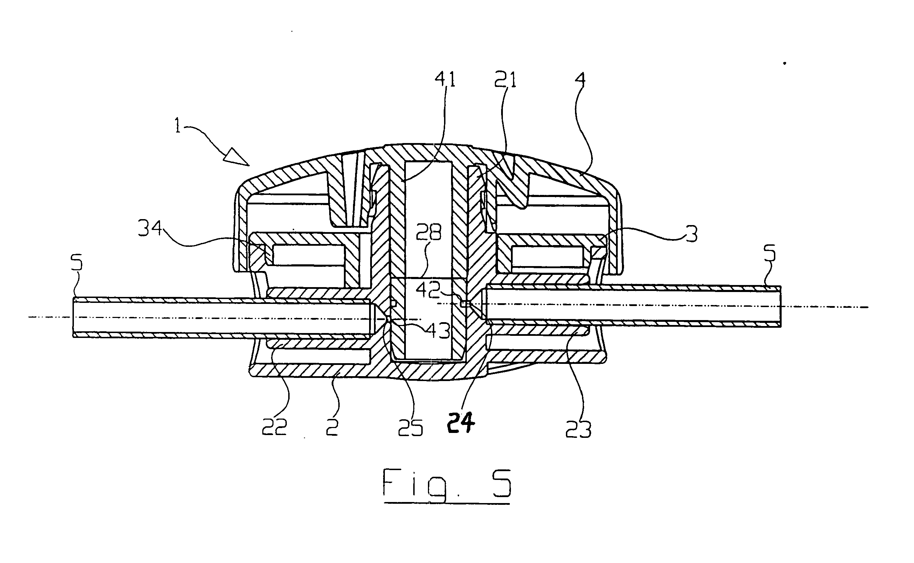

Fig. 5 zeigt einen Querschnitt durch das zusammengebaute Regelventil. Der Zusammenbau erfolgt so, daß als erstes der Einsatz 3 mit seinem Vorsprung 32 über den Ventilraum 21 des Gehäuses geschoben wird und dabei in Drehrichtung der Einsatz 3 so ausgerichtet wird, daß die Ausnehmungen 33 über die Anschlußstutzen 22 und 23 greifen. Aufgrund der unterschiedlichen axialen Höhe der Anschlußstutzen 22 und 23 und der entsprechenden unterschiedlichen Tiefe der Ausnehmungen 33 ist damit eine eindeutige Lage in Drehrichtung fixiert. Weiter greift der Zentrierbund 34 in den Rand des Gehäuses 2, womit der Einsatz 3 eine eindeutige Lage einnimmt.

Sodann wird das Drehteil 4 eingesetzt. Sein Ventilküken 41 greift dabei in die Öffnung des Ventilraumes 21 des Gehäuses 2. Durch den wechselseitigen Eingriff der Rastvertiefung 27 und der Rastnase 57 des Drehteiles erfolgt eine axiale Verriegelung. Die Nut 42 am Ventilküken 41 des Drehteiles 4 liegt dann korrekt gegenüber der Öffnung 24, so daß der Schlauch 5 des Anschlußstutzens 23 mit der Nut 42 in Strömungsverbindung steht. Durch den Kanal 44 ist die Nut 42 mit der Spiralnut 43 verbunden, womit eine Strömungsverbindung von der Öffnung 24 zur Öffnung 25 geschaffen ist, deren wirksamer Querschnitt von der Drehstellung des Drehteiles 4 abhängt.

Die Kante 28 definiert den Bereich zwischen dem Ventilraum 21 und dem Ventilküken 41, ab dem diesen beiden Teile dichtend aneinander liegen. Der Teil des Ventikükens 41 ab der Kante 28, der auch die beiden Nuten 42 und 43 trägt, ist dabei aus einem Material, das gegenüber dem Material des Ventilraumes 21 eine unterschiedliche Härte hat, damit dort ein guter dichtender Eingriff stattfindet. Die Dichtung wirkt alleine in der Mantelfläche des Ventilkükens 41. Die Stirnseite des Ventilkükens 41 hat dabei einen Abstand zum Boden der Ausnehmung des Ventilraumes 21, so daß auch für die axiale Zentrierung zwischen Gehäuse und Drehteil nur die Rastverbindung 27, 57 maßgeblich ist, ggf. zusammen mit einem Anschlag an der Stirnfläche des Ventilraumes 21. Ein geringes axiales Spiel hat auf die Dichtigkeit keinen Einfluß, wohl aber auf die Ausrichtung zwischen den Öffnungen 24 und 25 gegenüber den Nuten 42 und 43. Macht man die Öffnungen 24 und 25 etwas breiter als die Breite der Nuten 42 und 43, so tritt aber auch bei axialem Spiel keine Veränderung der Durchflußrate auf.

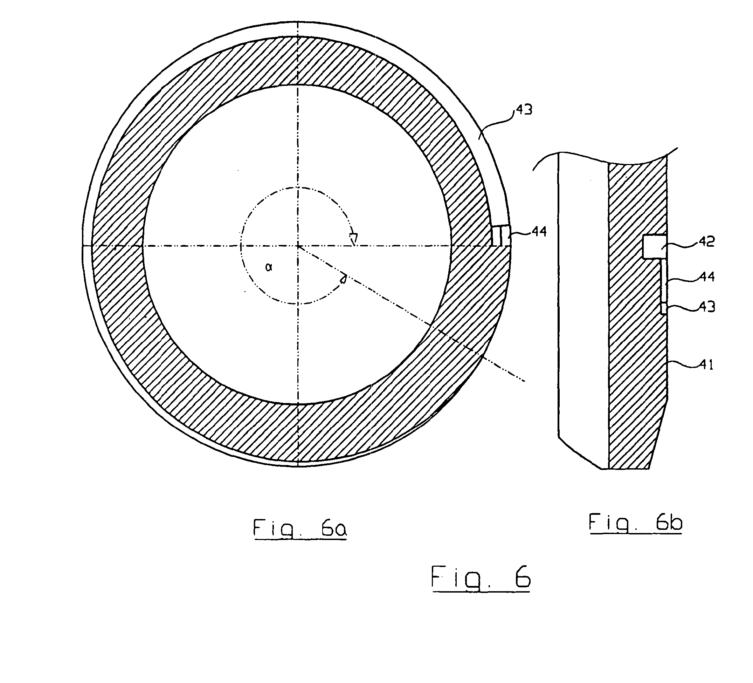

Fig. 6 zeigt noch einmal detaillierter die Nuten 42 und 43 und den Kanal 44. Die Nut 42 ist eine Ringnut mit konstantem Querschnitt, während die Nut 43 als Spiralnut bezeichnet wird und in Umfangsrichtung um den Winkel α verläuft und dabei abnehmende Tiefe bis zum Betrag Null hat. Über den Kanal 44 wird der Beginn der Nut 43 stets vollständig mit Flüssigkeit aus dem Kanal 42 versorgt. Je nach Drehstellung des Drehteiles 4 liegt eine unterschiedliche Tiefe der Nut 43 der Öffnung 25 gegenüber und bestimmt damit die Durchflußrate.

Aus obiger Beschreibung ist zu ersehen, daß die Montage sehr einfach und mit wenigen Handgriffen durchzuführen ist. Auch ist die spätere Bedienung des Regelventiles sehr einfach und kann mit etwas Geschick auch mit einer Hand durchgeführt werden. Über die Fenster 45, 46, 47 läßt sich die Durchflußrate für verschiedene Höhen des Behälters mit der Infusionsflüssigkeit bequem ablesen.

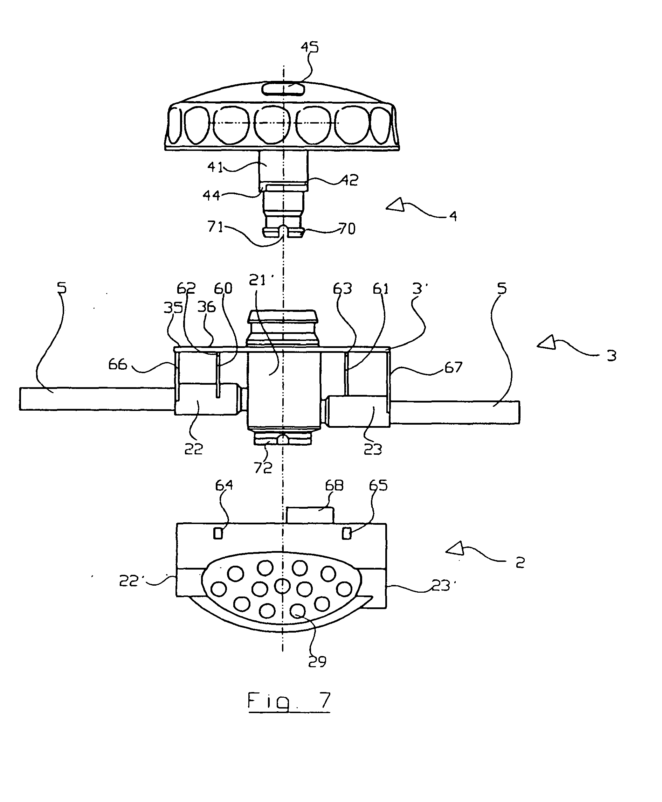

Das Ausführungsbeispiel der Figuren 7-11 unterscheidet sich von dem der Figuren 1-6 im wesentlichen durch folgende Abwandlungen, wobei zunächst auf Fig. 7 Bezug genommen wird. Der Einsatzkörper 3, der in das Gehäuse 2 eingesetzt wird, enthält bei dem Ausführungsbeispiel der Fig. 7 den Ventilraum 21, die beiden Anschlußstutzen 22 und 23 und die Ringscheibe, die hier mit dem Bezugszeichen 3' bezeichnet ist. Dieser komplette Einsatz 3 wird dann in das Gehäuse 2 eingesetzt. Die Ringscheibe 3' mit den aufgedruckten Skalen 35, 36 ist damit an den Ventilkörper angebunden und das Gehäuse 2 wurde vom Ventilkörper abgetrennt. Das Gehäuse 2 ist somit ein separates Spritzgußteil mit Grifflächen 29, das mit dem Einsatz 3 über eine Schnappverbindung verbunden wird. Weiter wurde bei diesem Ausführungsbeispiel der Drehanschlag für das Drehteil an den Außenrand des Gehäuses gelegt.

Die formschlüssige Verbindung (Einrastung bzw. Verschnappung) erfolgt einerseits zwischen dem Drehteil und dem Ventilkörper und andererseits zwischen dem Gehäuse und dem Ventilkörper.

Schließlich sind bei diesem Ausführungsbeispiel nur zwei Sichtfenster für das Ablesen der Skalen vorgesehen.

Soweit im Ausführungsbeispiel der Figuren 7-11 gleiche Bezugszeichen wie bei den Figuren 1-6 verwendet werden, bezeichnen die gleiche bzw. funktionell einander entsprechende Teile.

Es wird jetzt detailliert auf Fig. 7 Bezug genommen. Das Gehäuse 2 hat einen topfförmigen Grundkörper mit zwei seitlichen Ausnehmungen 22' und 23' zur Aufnahme der Anschlußstutzen 22 und 23 des Ventilkörpers 3, der von oben in das Gehäuse eingesetzt wird. Von den Anschlußstutzen 22 und 23 stehen scheibenförmige Stege 60 und 61 ab, die jeweils zwei Rastnasen 62, 63 aufweisen, welche in Rastöffnungen 64, 65 des Gehäuses 2 eingreifen können und damit den Ventilkörper 3 fest in dem Gehäuse 2 verankern. An den Stegen 60 und 61 ist die den Ventilraum 21 übergreifende ringförmige Scheibe 3' befestigt, die die abzulesenden Skalen trägt. Zusätzlich kann diese Scheibe noch über weitere Stege 66 und 67 an den Anschlußstutzen befestigt sein.

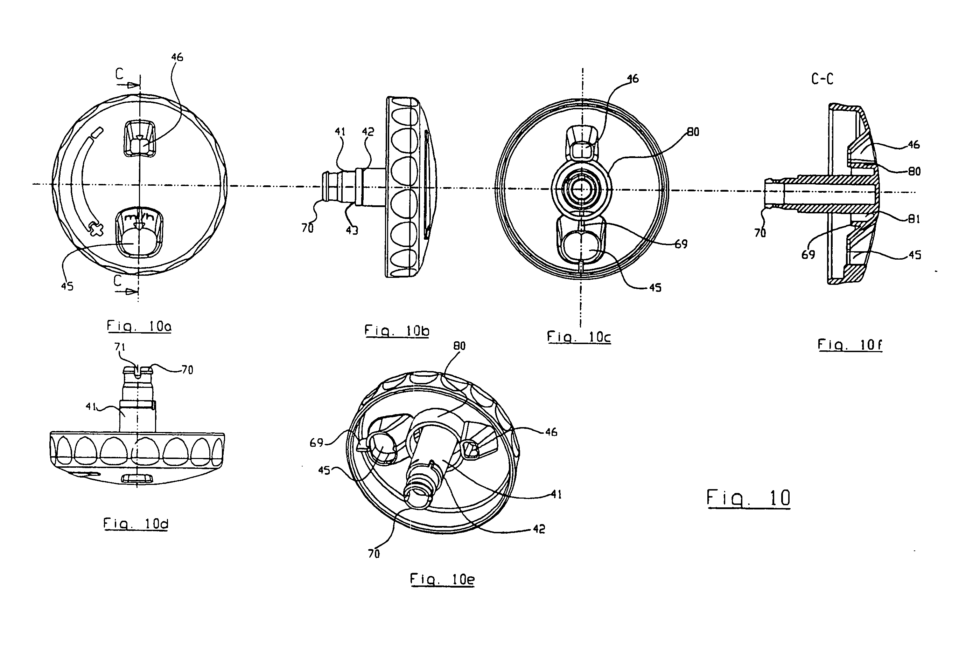

Am oberen Rand des Gehäuses 2 ist ein axial vorspringender Drehanschlag 68 vorgesehen, der mit einem Gegenanschlag 69 (Fig. 10) des Drehteiles zusammenwirkt.

Am freien Ende des Ventilkükens 41 ist eine umlaufende Rastnase 70 angebracht, die durch axiale Schlitze 71 federelastische Eigenschaften hat und beim Einführen des Ventilkükens in den Ventilraum 21 aus dessen offener Unterseite 72 herausragt und hinter der dort befindlichen Stirnseite einrastet.

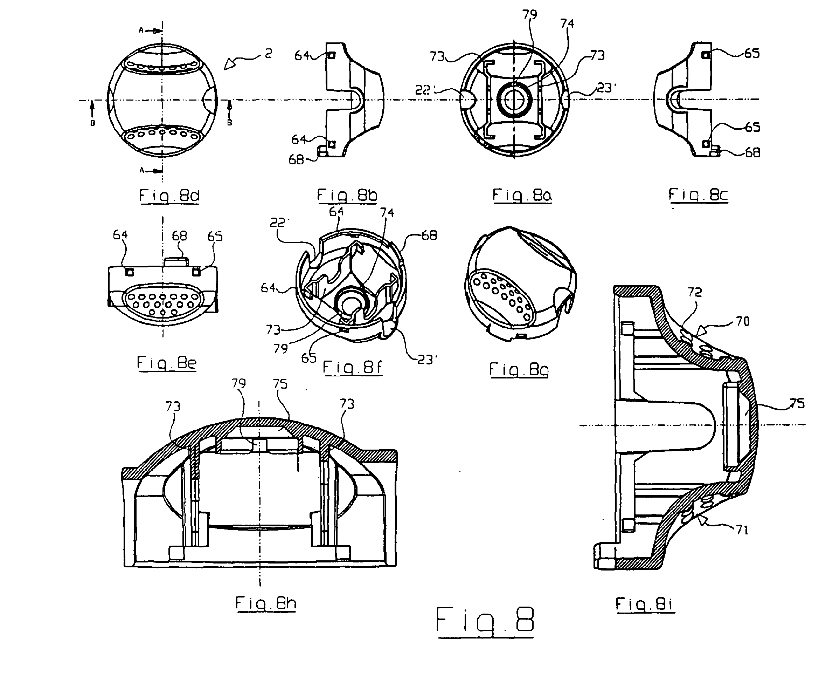

Fig. 8 zeigt das Gehäuse 2, und zwar in Draufsicht von oben (Fig. 8a), Seitenansichten von rechts und links (Fig. 8b und 8c), Ansicht von unten (Fig. 8d), Vorderansicht (Fig. 8e), perspektivischer Draufsicht (Fig. 8f), perspektivischer Ansicht von unten (Fig. 8g) sowie im Schnitt längs der Linie B-B (Fig. 8h) und längs der Linie A-A (Fig. 8i). An der Unterseite des Gehäuses sind Einbuchtungen 70 und 71 mit Griffnoppen 72 vorgesehen, über die das Gehäuse mit einer Hand gut und rutschfest ergriffen werden kann. Im inneren des Gehäuses sind Stege 73 und 74 vorgesehen zur Aufnahme des Einsatzteiles 3, wobei die Stege 73 an die Form der Anschlußstutzen 22 und 23 sowie der Stege 62, 63 und 66, 67 angepaßt sind, so daß das Einsatzteil 3 dort formschlüssig gehalten ist. Der Steg 74 im Gehäuse ist ringförmig und dient zur Zentrierung des Ventilraumes 21. Eine weitere Vertiefung 75 im Boden des Gehäuses dient zur Aufnahme des aus dem Ventilraum 21 herausragenden freien Endes des Ventilkükens 41.

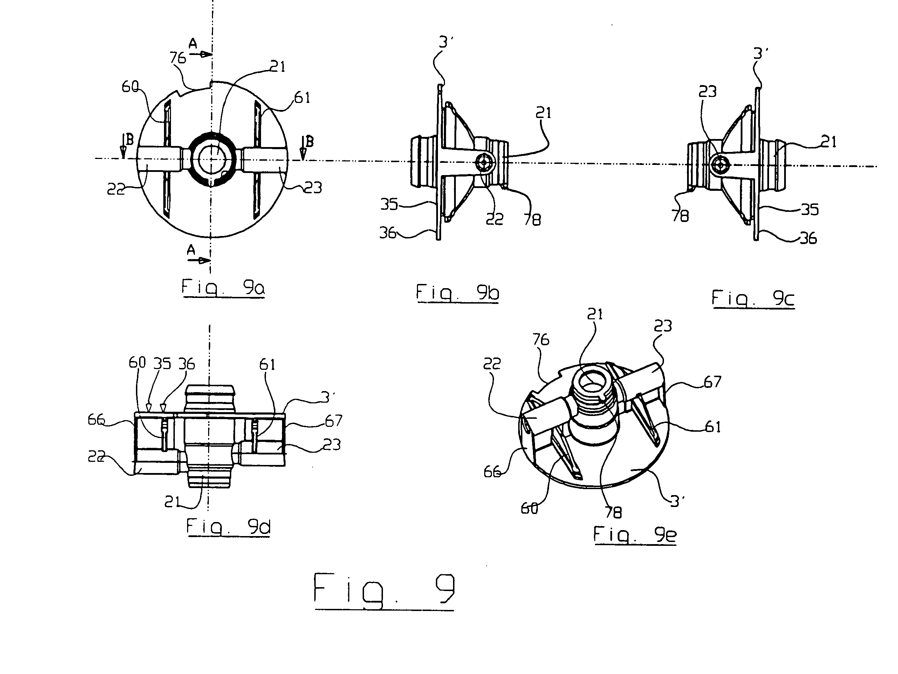

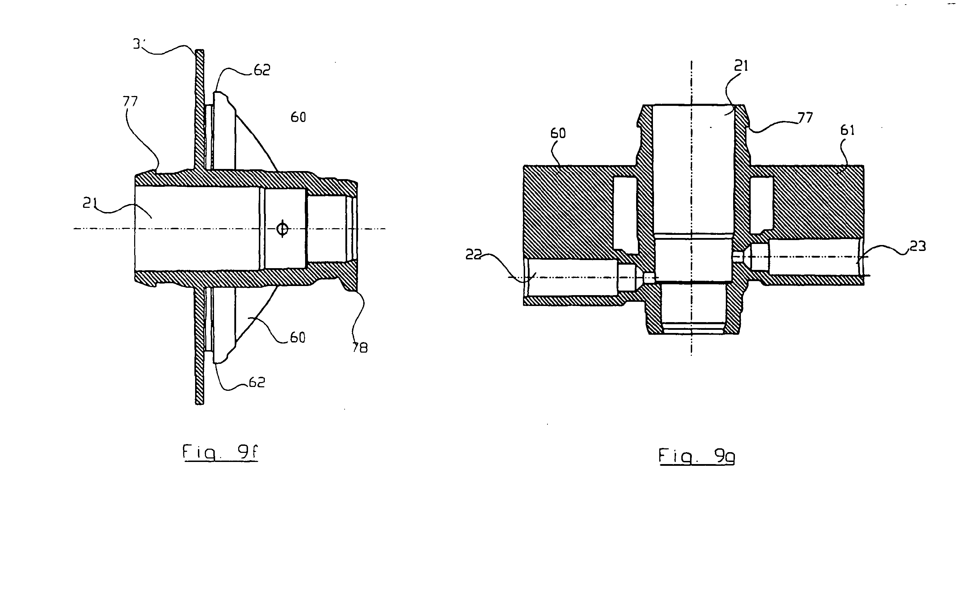

Fig. 9 zeigt den Ventilkörper 3 in einer Ansicht von unten (Fig. 9a), Seitenansichten von links und rechts (Fig. 9b und 9c), einer Vorderansicht (Fig. 9d), einer perspektivischen Ansicht schräg von unten (Fig. 9e) sowie Schnittansichten längs der Linie A-A der Fig. 9a (Fig. 9f) und längs der Linie B-B (Fig. 9g).

Diese Figuren sind mit den angegebenen Bezugszeichen im Zusammenhang mit der obigen Beschreibung der Fig. 7 aus sich heraus verständlich. Aus Fig. 9a und 9b ist noch zu erkennen, daß die Ringscheibe 3' an ihrem Außenumfang eine Ausnehmung 76 aufweist für den Durchtritt des Anschlages 68 des Gehäuses 2 (vgl. Fig. 7). Weiter ist zu erkennen, daß am oberen äußeren Rand des Ventilkörpers ein ringsumlaufender Vorsprung 77 vorhanden ist, der in eine weiter unten beschriebene Ausnehmung 81 des Drehteiles eingreift. Am unteren Ende des Ventilkörpers 21 ist ein Vorsprung 78 vorgesehen (vgl. Fig. 9b, 9e und 9f), der in eine Aussparung 79 (vgl. Fig. 8h) im Steg 74 des Gehäuses eingreift. Der Vorsprung 78 dient im wesentlichen als Anspritzpunkt beim Spritzgießen, um eine Freistrahlbildung zu vermeiden. Um diesen Vorsprung später im Gehäuse aufzunehmen, ist im Steg 74 des Gehäuses eine Aussparung 79 vorgesehen. Hierdurch wird zusätzlich eine Ausrichtung des Ventilkörpers gegenüber dem Gehäuse erreicht.

Fig. 10 zeigt das Drehteil 4 in Draufsicht von oben (Fig. 10a), Seitenansicht (Fig. 10b), Ansicht von unten (Fig. 10c), Vorderansicht (Fig. 10d), einer perspektivischen Ansicht schräg von unten (Fig. 10e) und einem Schnitt längs der Linie C-C der Fig. 10a (Fig. 10f). Das Drehteil ist auch hier topfförmig und hat zwei Fenster 45, 46 zum Ablesen der Skalen auf der Ringscheibe 3'. In den Figuren 10f und 10e ist der Anschlag 69 zu erkennen, der als Gegenanschlag zu dem Anschlag 68 des Gehäuses 2 (Fig. 7) dient und die Drehbegrenzung des Drehteiles bewirkt. Weiter ist am freien Ende des Ventilkükens 41 die Rastnase 70 zu erkennen, mit der das Drehteil am Ventilkörper 3 verriegelt wird. Rings um das Ventilküken 41 ist ein axial vorspringender Rand 80 zu sehen, der in radialem Abstand zu dem Ventilküken 41 verläuft, so daß dort ein Ringraum 81 gebildet wird, in den ein Ende des Ventilraumes 21 mit dem Vorsprung 77 eingreift, so daß das Drehteil hier gegenüber dem Ventilkörper 3 geführt bzw. gelagert ist.

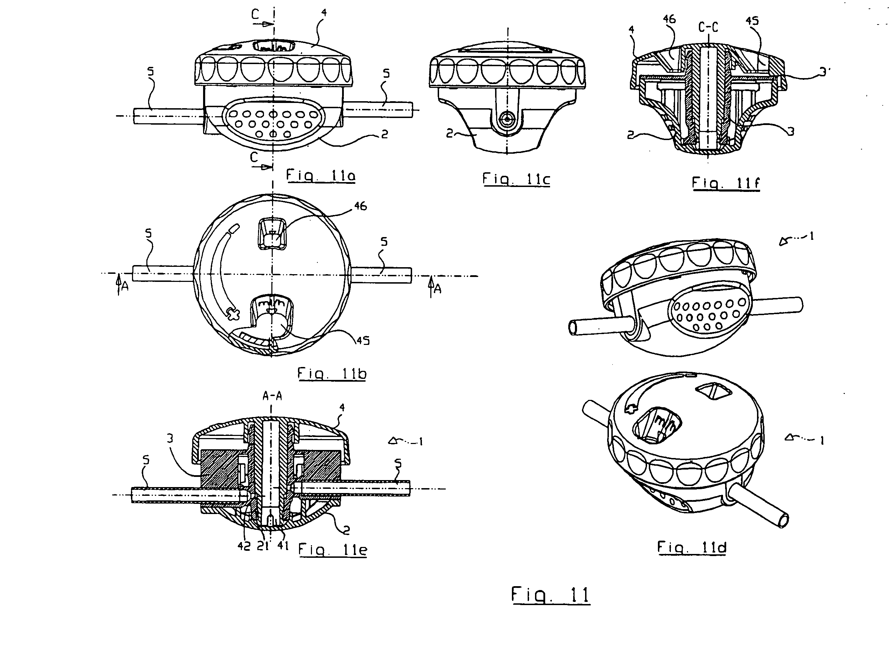

Fig. 11 zeigt das Regelventil im zusammengebauten Zustand, und zwar in Seitenansicht (Fig. 11a), Draufsicht (Fig. 11b), in einer um 90° gedrehten Seitenansicht (Fig. 11c), in zwei perspektivischen Ansichten schräg von oben und schräg von unten (Fig. 11d) sowie einem Schnitt längs der Linie A-A der Fig. 11b (Fig. 11e) und einem Schnitt längs der Linie C-C der Fig. 11a (Fig. 11f).

Die Schnittzeichnungen der Figuren 11e und 11f zeigen deutlich den zusammengebauten Zustand und wie die einzelnen Komponenten ineinander greifen bzw. aneinander angepaßt sind. Aufgrund der detaillierten Einzelteilzeichnungen und der dazu gehörigen Beschreibung ist eine weitere Erläuterung nicht erforderlich, da der Fachmann ohne weiteres Aufbau und Zusammenbau sowie Funktion der Einzelteile erkennt. Im Zusammenhang mit Fig. 11e ist jedoch noch zu erwähnen, daß im dort gezeigten Ausführungsbeispiel die zwei Ringnuten 42 und 43 des Ventilkükens 41 des ersten Ausführungsbeispieles der Fig. 4 etwas anders ausgebildet sind. Die eine Nut 42 mit konstanter Tiefe und konstantem Querschnitt, die auch als Ringraum mit konstanter Tiefe und konstantem Querschnitt bezeichnet werden kann, wird hier nicht als Nut im Ventilküken realisiert sondern durch zwei Stufen am Ventilküken 41 und am Ventilraum 21, die axial gegeneinander versetzt angeordnet sind, und zwar gerade um den Durchmesser des in den Ventilraum mündenden einen Anschlusses.

## Patentansprüche

1. Regelventil zum Einstellen des Volumenstromes bei einem Infusions- oder Transfusionsvorgang mit
- einem Gehäuse (2),
- einem Einlaß- und einen Auslaßanschlußstutzen (22, 23), die in einen hohlzylindrischen Ventilraum (21) münden,
- einem Drehteil (4), das einen topfartigen Grundkörper und ein davon abstehendes zylindrisches Ventilküken (41) aufweist, das außenseitig dichtend in den Ventilraum (21) einsetzbar ist und an seinem Außenumfang mindestens eine den Anschlußstutzen (22, 23) gegenüberliegende Nut (42, 43) aufweist, die die Anschlußstutzen (22, 23) strömungsmäßig miteinander verbindet und in Umfangsrichtung einen sich ändernden Querschnitt aufweist,
**gekennzeichnet durch**
eine Kreisringscheibe (3'), die den Ventilraum (21) übergreift und die an ihrer zum Drehteil (4) weisenden Seite mehrere auf konzentrischen Kreisen liegende Markierungen (35, 36, 37) aufweist, und
daß das Drehteil (4) mehrere Fenster (45, 46, 47) aufweist, die in Umfangsrichtung und in radialer Richtung gegeneinander versetzt angeordnet sind und jeweils einer der Markierungen (35, 36, 37) gegenüberliegen.

2. Regelventil nach Anspruch 1, **dadurch gekennzeichnet, daß** die Kreisringscheibe (3') einen axial abstehenden ringförmigen Zentrierbund (34) aufweist, der in das Gehäuse (2) eingreift.

3. Regelventil nach Anspruch 1 oder 2, **dadurch gekennzeichnet,**
**daß** die Kreisringscheibe (3') einen zylindrischen Vorsprung (32) aufweist, der an seiner Stirnseite einander gegenüberliegende Ausnehmungen (33) aufweist, die die Anschlußstutzen (22, 23) des Gehäuses (2) übergreifen.

4. Regelventil nach einem der Ansprüche 1 bis 3, **dadurch gekennzeichnet,**
**daß** an der Außenseite des Ventilraumes (21) ein Drehbegrenzungsanschlag (50) vorgesehen ist, der mit einem Drehbegrenzungsanschlag (51) am Drehteil (4) zusammenwirkt.

5. Regelventil nach einem der Ansprüche 1 bis 4, **dadurch gekennzeichnet,**
**daß** der Ventilraum (21) an seiner Außenseite eine Rastvertiefung (27) aufweist, und daß das Drehteil (4) konzentrisch zum Ventilküken (41) einen zylindrischen Vorsprung mit mindestens einer Rastnase (57) aufweist, die in die Rastvertiefung (27) eingreift.

6. Regelventil nach einem der Ansprüche 1 bis 5, **dadurch gekennzeichnet,**
**daß** die Längsachsen der Anschlußstutzen (22, 23) bezogen auf die Drehachse (26) des Regelventiles axial gegeneinander versetzt sind.

7. Regelventil nach Anspruch 6, **dadurch gekennzeichnet, daß** das Ventilküken (41) an seinem Außenumfang zwei Nuten (42, 43) aufweist, von denen die eine dem Einlaß-Anschlußstutzen und die andere dem Auslaß-Anschlußstutzen zugeordnet ist, wobei die beiden Nuten (42, 43) durch einen axialen Kanal (44) strömungsmäßig miteinander verbunden sind.

8. Regelventil nach einem der Ansprüche 1 bis 7, **dadurch gekennzeichnet,**
**daß** der Innenraum des Ventilraumes (21) eine Stufe (28) aufweist, ab welcher zwischen Ventilraum (21) und Ventilküken (41) ein dichtender Eingriff stattfindet.

9. Regelventil nach Anspruch 1, **dadurch gekennzeichnet, daß** die Kreisringscheibe (3'), der Ventilraum (21) und die Anschlußstutzen (22, 23) als ein Einsatzteil (3) ausgebildet sind, das in das Gehäuse (2) einsetzbar ist.

10. Regelventil nach Anspruch 9, **dadurch gekennzeichnet, daß** am oberen äußeren Rand des Gehäuses (2) ein Drehanschlag (68) und am Boden des Drehteiles (4) ein Gegenanschlag (69) vorgesehen sind.

11. Regelventil nach Anspruch 10, **dadurch gekennzeichnet, daß** der Einsatz (3) mit Rastnasen (62, 63) in Öffnungen (64, 65) des Gehäuses (2) verriegelbar ist.

12. Regelventil nach Anspruch 11, **dadurch gekennzeichnet, daß** das Drehteil mit integriertem Ventilküken (41) mittels eines Rastvorsprunges (70) am Ventilraum (21) verriegelbar ist.

## Claims

1. Regulating valve for adjusting the volume flow in an infusion or transfusion procedure said regulating valve comprising
- a housing (2),
- an inlet connecting piece (22) and an outlet connecting piece (23) opening into a hollow cylindrical valve chamber (21),
- a rotating piece (4) that has a cup-shaped foundation and a cylindrical valve plug (41) extending therefrom, wherein said cylindrical valve plug is inserted into the valve chamber (21) sealing the valve chamber, where said rotating piece has, on a periphery, at least one groove (42, 43) that is opposite the connecting pieces (22, 23) and which constrictingly connects the connecting pieces (22, 23) in terms of flow, where said rotating piece has a changing cross-section in the peripheral direction,
**characterized by**
an annular disc (3') which overlaps the valve chamber (21) and has, on a side facing the rotating piece (4), a number of markings (35, 36, 37) lying on concentric circles, and
in that the rotating piece (4) has a number of windows (45, 46, 47) that are in an offset arrangement with respect to each other in the peripheral and radial directions, where each window is lying opposite a marking (35, 36, 37).

2. Regulating valve according to claim 1, **characterized in that** the annular disk (3') has, at an axial distance, an annular centering collar (34) which engages the housing (2).

3. Regulating valve according to claim 1 or 2, **characterized in that** the annular disk (3') has a cylindrical projection (32) which has, on its face, opposing recesses (33) which overlap the connecting pieces (22, 23) of the housing (2).

4. Regulating valve according to one of claims 1 to 3, **characterized in that** on the outside of the valve chamber (21) a rotation limiting stop (50) is provided cooperating with a rotation limiting stop (51) on the rotating piece (4).

5. Regulating valve according to one of claims 1 to 4, **characterized in that** the valve chamber (21) has, on its outside, a catch recess (27) and **in that** the rotating piece (4) has, concentric to the valve plug (41) a cylindrical projection with at least one detent (57) which engages the catch recess (27).

6. Regulating valve according to one of claims 1 to 5, **characterized in that** the longitudinal axes of the connecting pieces (22, 23) are, in relation to the axis of rotation (26) of the regulating valve, axially offset from one another.

7. Regulating valve according to claim 6, **characterized in that** the valve plug (41) has, on its outer periphery, two grooves (42, 43), one of which is assigned to the inlet connecting piece and the other to the outlet connecting piece, the two grooves (42, 43) being connected to each other in terms of flow by an axial channel (44).

8. Regulating valve according to one of claims 1 to 7, **characterized in that** the interior of the valve chamber (21) has a step (28) after which a sealing engagement takes place between the valve chamber (21) and the valve plug (41).

9. Regulating valve according to claim 1, **characterized in that** the annular disc (3'), the valve chamber (21) and the connecting pieces (22, 23) are designed as an insert part (3) which can be inserted into the housing (2).

10. Regulating valve according to claim 9, **characterized in that** at the upper outer edge of the housing (2) a rotating stop (68) is provided and a counter-stop (69) is provided at the base of the rotating piece (4).

11. Regulating valve according to claim 10, **characterized in that** the insert (3) with detents (62, 63) can be locked in the openings (64, 65) of the housing (2).

12. Regulating valve according to claim 11, **characterized in that** the rotating piece with integrated valve plug (41) can be locked on the valve chamber (21) by means of a detent projection (70).

## Revendications

1. Valve pour contrôler le débit dans un procédé de perfusion ou de transfusion, avec
- un boîtier (2),
- un raccord d'admission et un raccord de sortie (22, 23), qui débouchent dans une chambre de valve creuse cylindrique (21),
- une pièce rotative (4), qui présente un élément de base en forme de pot et un boisseau de valve cylindrique (41) qui saille dudit élément de base, est insérable dans la chambre de valve (21) de façon étanche à l'extérieur et présente sur sa circonférence externe au moins une rainure (42, 43) opposée aux raccords (22, 23), laquelle assure une liaison d'écoulement entre lesdits raccords (22, 23) et présente dans le sens de la circonférence une section transversale qui varie,
**caractérisée par**
un disque annulaire (3'), qui recouvre la chambre de valve (21) et qui présente sur sa face orientée vers la pièce rotative (4) plusieurs repères (35, 36, 37) disposés sur des cercles concentriques, et en ce que
la pièce rotative (4) présente plusieurs fenêtres (45, 46, 47), qui sont décalées les unes par rapport aux autres dans le sens de la circonférence et dans le sens radial et qui sont respectivement opposées à l'un des repères (35, 36, 37).

2. Valve selon la revendication 1, **caractérisée en ce que**
le disque annulaire (3') présente un collet de centrage annulaire (34), qui saille dans le sens axial et s'engrène dans le boîtier (2).

3. Valve selon la revendication 1 ou 2, **caractérisée en ce que**
le disque annulaire (3') présente une saillie cylindrique (32), qui présente sur sa face terminale des encoches (33) opposées l'une à l'autre, lesquelles recouvrent les raccords (22, 23) du boîtier (2).

4. Valve selon l'une des revendications 1 à 3, **caractérisée en ce que**
sur la face externe de la chambre de valve (21) est prévue une butée de limitation de rotation (50), qui coopère avec une butée de limitation de rotation (51) prévue sur la pièce rotative (4).

5. Valve selon l'une des revendications 1 à 4, **caractérisée en ce que**
la chambre de valve (21) présente sur sa face externe un creux d'arrêt (27), et la pièce rotative (4) présente concentriquement par rapport au boisseau de valve (41) une saillie cylindrique munie d'au moins un taquet d'arrêt (57), lequel s'engrène dans le creux d'arrêt (27).

6. Valve selon l'une des revendications 1 à 5, **caractérisée en ce que**
les axes longitudinaux des raccords (22, 23) sont décalés l'un par rapport à l'autre dans le sens axial relativement à l'axe de rotation (26) de la valve.

7. Valve selon la revendication 6, **caractérisée en ce que**
le boisseau de valve (41) présente sur sa circonférence externe deux rainures (42, 43), dont l'une est affectée au raccord d'admission et l'autre au raccord de sortie, un canal axial (44) assurant une liaison d'écoulement entre lesdites deux rainures (42, 43).

8. Valve selon l'une des revendications 1 à 7, **caractérisée en ce que**
l'intérieur de la chambre de valve (21) présente un gradin (28), à partir duquel une action d'étanchéité a lieu entre la chambre de valve (21) et le boisseau de valve (41).

9. Valve selon la revendication 1, **caractérisée en ce que**
le disque annulaire (3'), la chambre de valve (21) et les raccords (22, 23) sont conçus comme un insert (3) insérable dans le boîtier (2).

10. Valve selon la revendication 9, **caractérisée en ce que**
sur le bord extérieur supérieur du boîtier (2) est prévue une butée de rotation (68) et sur le fond de la pièce rotative (4) une contre-butée (69).

11. Valve selon la revendication 10, **caractérisée en ce que**
l'insert (3) est verrouillable dans des orifices (64, 65) du boîtier (2) par des taquets d'arrêt (62, 63).

12. Valve selon la revendication 11, **caractérisée en ce que**
la pièce rotative avec boisseau de valve intégré (41) est verrouillable sur la chambre de valve (21) au moyen d'une saillie d'arrêt (70).
